Europäisches Patentamt

European Patent Office

Office européen des brevets

(19)

(11) Publication number: **0 430 542 A2**

(12)                         # EUROPEAN PATENT APPLICATION

(21) Application number: **90312622.5**

(22) Date of filing: **20.11.90**

(51) Int. Cl.⁵: **B29D 23/22, F16L 11/00, B29C 53/56**

(30) Priority: **29.11.89 JP 307616/89**

(43) Date of publication of application:
**05.06.91 Bulletin 91/23**

(84) Designated Contracting States:
**DE FR GB**

(71) Applicant: **KABUSHIKI KAISHA MACHIDA SEISAKUSHO**
**13-8, Honkomagome 6-chome Bunkyo-ku**
**Tokyo (JP)**

(72) Inventor: **Kato, Kenichi, 328-16, Kokuki, Shiraoka-Machi**
**Minamisaitama-Gun**
**Saitama-Ken (JP)**
Inventor: **Nobuhara, Toshihide, 16-15-202, Shiratori 2-chome**
**Katsushika-Ku**
**Tokyo (JP)**

(74) Representative: **Votier, Sidney David et al**
**CARPMAELS & RANSFORD 43, Bloomsbury Square**
**London WC1A 2RA (GB)**

(54) **Method of producing flexible tube.**

(57)    There is disclosed a method of producing a flexible tube (10 ; 10A). A braid (12) is fitted on an outer periphery of an elongated core member (20) of a cylindrical shape. Then, a soft resin layer (13 ; 13A) is formed on an outer periphery of the braid, and the resin layer and the braid are joined together to form a sheath (15 ; 15A). The core member is removed from the sheath. Subsequently, a flex (11) in the form of a spirally-wound metal strip is inserted into the sheath. The core member can be removed from the sheath by dipping the core member in a dissolvable solution (22).

Fig.3

EP 0 430 542 A2

# METHOD OF PRODUCING FLEXIBLE TUBE

## BACKGROUND OF THE INVENTION

This invention relates to a method of producing a flexible tube for use, for example, in an insertion portion of an endoscope or a catheter.

Generally, a flexible tube used in an endoscope comprises a flex formed by winding a metal strip into a spiral shape, a braid provided around the outer periphery of the flex, and a resin layer formed on the outer periphery of the braid. The braid and the resin layer are joined together to form a sheath. The flex and the sheath are connected together only at their opposite ends.

The above flexible tube is so flexible as to be bent. The flex resists a compressive force acting on the flexible tube in a radial direction of the flexible tube, thus preventing the flexible tube from being crushed flat. The braid resists a tensile force acting on the flexible tube in an axial direction of the flexible tube, and also resists a torsional force acting on the flexible tube. Thus, the braid prevents the elongation and torsion of the flexible tube. The resin layer resists a compressive force acting on the flexible tube in an axial direction of the flexible tube, thus preventing an axial contraction of the flexible tube. The resin layer also imparts liquid-sealing properties to the flexible tube.

In the above sheath, the braid and the resin layer reinforce each other. If the resin layer and the braid are separated from each other, wrinkles develop on the resin layer at an inner side of a bent portion of the flexible tube when the flexible tube is bent. Therefore, repeated bending of the flexible tube may cause the resin layer to be torn because of fatigue. Also, the braid may be cut because of fatigue. Therefore, the braid and the resin layer must be joined together firmly.

On the other hand, when the flexible tube is bent, the interval or distance between any adjacent turns of the flex becomes greater at the outer side of the bent flexible tube, and becomes smaller at the inner side thereof. Therefore, in order to reduce the bending resistance of the flexible tube, it is necessary that each turn of the flex should be movable relative to the sheath.

An ordinary conventional method of producing the above flexible tube will now be described. First, the braid is fitted on the flex. Then, an adhesive is coated on and impregnated in the braid. Then, the resin layer is formed on the outer periphery of the braid either by fitting a heat-shrinkable tube on the braid or by extrusion of a resin. In this case, the adhesive layer is recognized as an inner resin layer.

Another method without the use of an adhesive may be used in which the braid, fitted on the flex, is impregnated with a resin by dipping, and then the resin is dried to form the resin layer. In this case, the braid is embedded in the inside portion of the resin layer.

In the method using the adhesive, in order to meet the above first requirement (that is, the firm connection between the braid and the resin layer), the adhesive must be adequately penetrated into the braid. In this case, the adhesive reaches to the outer peripheral surface of the flex. As a result, each turn of the flex is adhesively bonded to the sheath, and therefore can not move relative to the sheath, thus failing to meet the above second requirement. In contrast, if the amount of impregnation of the adhesive in the braid is reduced in order to meet the second requirement, then the strength of bonding between the resin layer and the braid is decreased, thus failing to meet the first requirement.

In the method without the use of an adhesive, when it is intended to adequately impregnate the liquid-state resin into the braid, part of this resin intrudes into the spaces between the turns of the flex, and then is solidified to form convex portions projecting radially inwardly, thus failing to meet the second requirement. In contrast, if the amount of impregnation of the resin into the braid is reduced in order to meet the second requirement, then the strength of connection between the braid and the resin is decreased, thus failing to meet the first requirement.

Next, reference is now made to the prior art. Japanese Laid-Open (Kokai) Patent Application No. 195538/83 discloses a technique in which a non-adhesive layer is formed on an outer peripheral surface of a flex, thereby preventing the flex from being adhesively bonded to a sheath even if an adhesive reaches the outer peripheral surface of the flex. With this method, however, part of the adhesive intrudes into the spaces between the adjacent turns of the flex, and then is solidified to form convex portions projecting radially inwardly. These convex portions prevent the turns of the flex from moving relative to the sheath.

Japanese Laid-Open Patent Application No. 163330/83 discloses a method in which a resin layer is formed by extrusion. In this method, where an adhesive is not used, a molten resin intrudes into the spaces between turns of a flex, and then is solidified to form convex portions projecting radially inwardly. These convex portions prevent the turns of the flex from moving relative to a sheath.

Japanese Laid-Open Patent Application No. 149124/84 discloses means for preventing an adhesive, penetrated into a braid, from reaching a flex. More specifically, a thin strip or band of a resin is wound on the outer periphery of the flex. Japanese Laid-Open Patent Application No. 137031/84 discloses a technique in which a thin tube is interposed between a flex and a braid. This tube prevents an adhesive from reaching the flex. In these cases, however, there is encountered a problem that upon repeated bending of the flexible tube, the strip or the tin tube may be ruptured or damaged by the frictional contact of the strip or the tin tube with the turns of the flex.

Japanese Laid-Open Patent Application No. 230614/85 discloses a method in which an elongated core member of synthetic rubber is used. A flex is fitted on the outer periphery of the core member, and a braid is fitted on the outer periphery of the flex. The braid is impregnated with an adhesive, and a resin layer is formed on the outer periphery of the braid. In this method, part of the adhesive intrudes into the spaces between the turns of the flex, and then is solidified to form convex portions projecting radially inwardly. The height of these convex portions is generally equal to the thickness of the flex, and the convex portions prevent the turns of the flex from moving relative to a sheath. This prior patent application does not describe how the core member is removed from the sheath.

Japanese Patent Publication No. 11011/88 discloses the prior art using an elongated core member. A braid is fitted on the core member. The braid is impregnated with an adhesive of a resin. Further, a tube is fitted on the braid, and is heat-shrunk to be brought into intimate contact with the braid. The adhesive resin is compressed by the heat-shrunk tube, so that the adhesive resin is sufficiently introduced into the meshes of the braid and is cured to form an elastic resin layer. Thus, it is intended to obtain a sheath having a smooth inner peripheral surface. This method is to produce a flexible tube having no flex. This prior patent publication does not described how the core member is removed from the sheath.

## SUMMARY OF THE INVENTION

It is an object of this invention to provide a method of producing a flexible tube which is so flexible as to be easily bent, and has a high durability.

According to one aspect of the present invention, there is provided a method of producing a flexible tube comprising the steps of :

(a) fitting a braid on an outer periphery of an elongated core member of a cylindrical shape ;

(b) forming a soft resin layer on an outer periphery of the braid, and joining the resin layer and the braid together to form a sheath ;

(c) removing the core member from the sheath ; and

(d) subsequently inserting a flex into the sheath, the flex being in the form of a spirally-wound metal strip.

According to another aspect of the invention, there is provided a method of producing a flexible tube comprising the steps of :

(a) fitting a braid on an outer periphery of an elongated core member of a cylindrical shape, the core member being made of a dissolvable material ;

(b) forming a soft resin layer on an outer periphery of the braid, and joining the resin layer and the braid together to form a sheath ; and

(c) removing the core member from the sheath by dipping the core member in a dissolving solution.

## BRIEF DESCRIPTION OF THE DRAWINGS

Fig. 1 is a side-elevational view of an endoscope incorporating a flexible tube provided in accordance with the present invention ;

Fig. 2 is a side-elevational view of the flexible tube ;

Fig. 3 is an enlarged cross-sectional view of a portion of the flexible tube ;

Fig. 4 is a view similar to Fig. 3, but showing a modified flexible tube ; and

Figs. 5 to 8 are views illustrative of the sequence of the formation of the flexible tube.

## DESCRIPTION OF THE PREFERRED EMBODIMENTS OF THE INVENTION

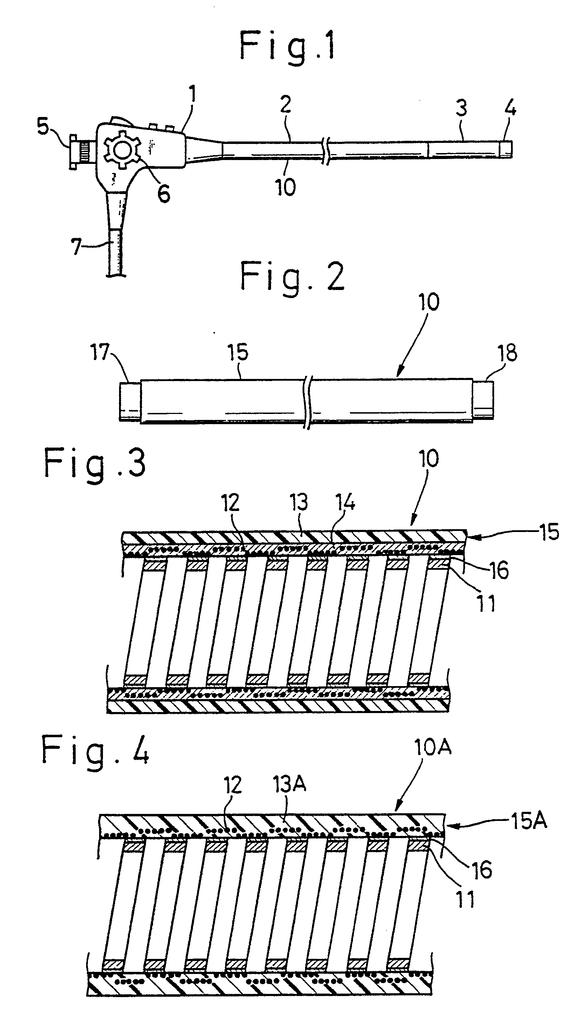

Before describing a flexible tube-producing method of the present invention, an endoscope and a flexible tube used in the endoscope will first be described with reference to Figs. 1 to 4.

As shown in Fig. 1, the endoscope comprises a body 1, an insertion portion 2 extending from one end of the body 1, a bending portion 3 extending from a distal end of the insertion portion 2, and a rigid portion 4 provided at a distal end of the bending portion 3. An ocular portion 5 is formed on the other end of the body 1, and is optically connected to an inspection window, formed on the rigid portion 4, via an optical fiber bundle and so on. A manipulation dial 6 for remote control of the bending portion 3 via wires is mounted on the peripheral wall of the body 1. A cable 7 is connected at one end to the body 1, and is connected at the other end to a light source. Light from the light source is fed to an illumination window, formed on the rigid portion 4, via an optical fiber bundle passing through the cable 7, the body 1, the insertion portion 2 and the bending portion 3, and the light is further emitted from the illumination window.

The insertion portion 2 has a flexible tube 10 shown in Figs. 2 and 3. The above-mentioned optical fiber bundles, the wires and etc., are passed through the flexible tube 10.

The flexible tube 10 comprises a flex 11 formed by winding a metal strip into a spiral shape, a braid 12 mounted around the outer periphery of the flex 11, a soft resin layer 13 mounted around the outer periphery of the braid 12. The braid 12 is embedded in an adhesive layer 14, and the braid 12 and the resin layer 13 are joined together through the adhesive layer 14. The braid 12, the resin layer 13 and the adhesive layer 14 jointly constitute a sheath 15 which is so flexible as to be bent. The adhesive layer 14 is made of an elastic resin, and can be recognized as an inner resin layer. In this embodiment, the braid 12 is made of narrow wires of steel, copper, a copper alloy or the like. A lubricating layer 16 is formed on the outer peripheral surface of the flex 11. The outer diameter of the flex 11 in its natural condition is slightly greater than the inner diameter of the sheath 15, and therefore the flex 11 is held in contact with the inner peripheral surface of the sheath 15 with a slight resilient force.

With respect to the dimensions of the flexible tube 10 shown in Fig. 3, the sheath 15 has an inner diameter of 8 mm and an outer diameter of 9.4 mm. The thickness of the braid 12 is 0.2 mm, and the thickness of the adhesive layer 14 is almost the same as the thickness of the braid 12. The thickness of the resin layer 13 is 0.5 mm, and the thickness of the flex 11 is 0.2 mm, and the thickness of the lubricating layer 16 is 30 µm.

As shown in Fig. 2, a pair of rings 17 and 18 of metal are attached to the opposite ends of the flexible tube 10. Each of the rings 17 and 18 has an inner diameter slightly greater than that of the sheath 15, and has an outer diameter slightly smaller than that of the sheath 15. The opposite ends of the braid 12, as well as the opposite ends of the flex 11, project respectively from the opposite ends of the adhesive layer 14 and also project respectively from the opposite ends of the resin layer 13. The opposite ends of the braid 12 as well as the opposite ends of the flex 11 are soldered respectively to the inner peripheral surfaces of the rings 17 and 18. The ring 17 is received in the body 1 and fixed thereto, and the ring 18 is connected to the proximal end of the bending portion 3.

Fig. 4 shows a modified flexible tube 10A. A resin layer 13A and a braid 12, which jointly constitute a sheath 15A of the flexible tube 10A, are directly joined together, with no adhesive layer interposed therebetween. Namely, the braid 12 is embedded in the inside portion of the resin layer 13. The other parts are the same as those of the flexible tube 10 of Fig. 3, and are designated by identical reference numerals, respectively, and therefore explanation of those corresponding parts is omitted.

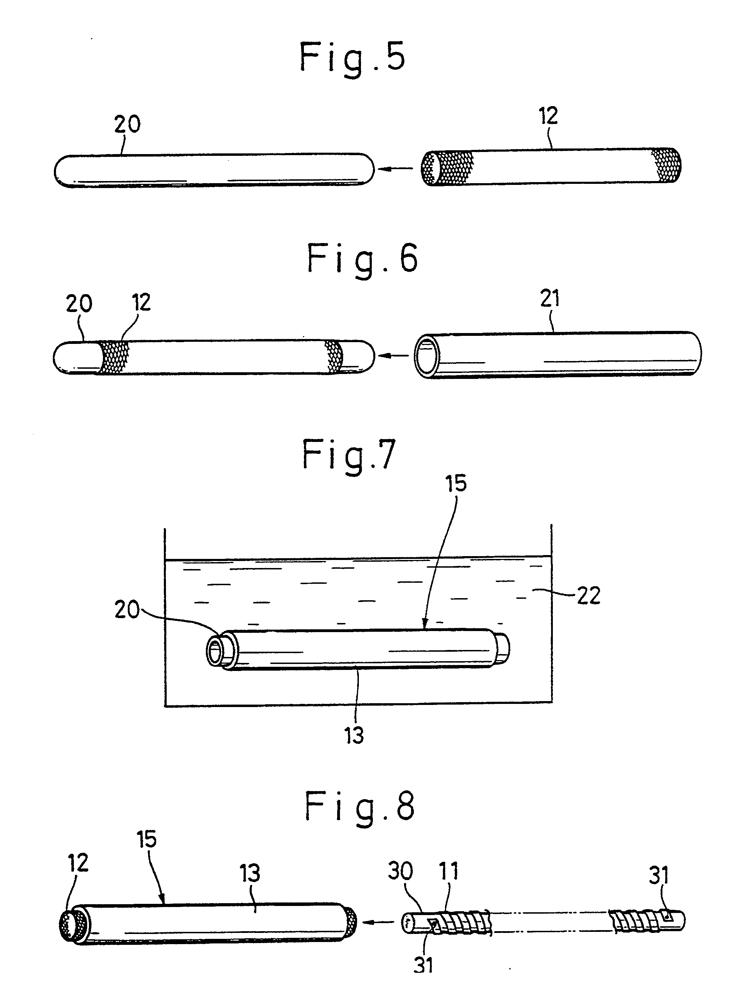

The basic construction of each of the flexible tubes 10 and 10A shown respectively in Figs. 3 and 4 is similar to a conventional construction. A method of producing the flexible tube 10 of Fig. 3 will now be described. As shown in Fig. 5, first, there is prepared a core member 20 in the form of a straight hollow cylindrical pipe. The core member 20 is made, for example, of a glass material which is dissolvable in an acid. The outer diameter of the core member 20 is slightly smaller than the outer diameter of the flex 11 in its natural condition. The opposite ends of the core member 20 are closed.

As shown in Fig.5, the braid 12 is fitted on the core member 20, and then the braid 12 is wiped or rubbed by rollers to be brought into intimate contact with the outer peripheral surface of the core member 20. Then, the braid 12 is fastened at its opposite ends to the core member 20 by tapes or the like. Instead of using the rollers, the braid 20 may be pulled axially of the core member 20 to be brought into intimate contact with the core member 20.

Then, a hot-melt type adhesive in a liquid state is applied to the outer peripheral surface of the braid 12 by coating, spraying or the like, so that the adhesive is impregnated into the braid 12. The thus impregnated adhesive is solidified or set to form an adhesive layer, and the braid 12 is embedded in this adhesive layer. The adhesive layer is not formed on the opposite end portions of the braid 12.

Then, as shown in Fig. 6, a heat-shrinkable tube 21 made, for example, of a polyurethane resin is fitted on the braid 12. Then, the tube 21 is heated to be shrunk radially, so that the tube 21 is intimately fitted on the braid 12. At this time, the adhesive layer is again melted by the heat applied to the tube 21, and bonds the tube 21 and the braid 12 together. Preferably, the above adhesive is a polyester-type hot melt agent which can be relatively satisfactorily bonded adhesively to both the tube 21 of a polyurethane resin and the braid 12 of metal. The adhesive, when cured or set, forms the adhesive layer 14 shown in Fig. 3, and the cured tube 21 serves as the resin layer 13. Thus, the sheath 15 is formed. The opposite end portions of the braid 12 do not have the adhesive layer, and therefore are not bonded to the tube 21.

Then, as shown in Fig. 7, the closed opposite ends of the core member 20 is cut to be opened, and then the above sheath assembly is dipped in a dissolving solution 22 of an acid, so that the core member 20 is dissolved. Since the core member 20 has a pipe-shape, the core member 20 begins to be dissolved from its inner peripheral surface. This enhances the dissolving efficiency. Preferably, the dissolving solution 22 is circulated in order to promote the dissolving of the core member 20. Naturally, the dissolving solution 22 is of the type which dissolves only the core member 20 and does not dissolve the braid 12, the adhesive layer 14 and the resin layer 13. In this embodiment, nitric acid is used as the dissolving solution. Upon dissolving of the core member 20, the sheath 15 remains.

In the case where the core member 20 is in the form of a pipe so that the dissolving of the core member 20 can begin from its inner peripheral surface, it is particularly necessary that before the dissolving, foreign matters of a non-dissolving nature should not adhere to the inner peripheral surface of the core member 20. For this reason, in this embodiment, the opposite ends of the core member 20 are closed beforehand.

Then, as shown in Fig. 8, the opposite end portions of the resin layer 13 which are not bonded to the braid 12 are cut so as to expose the opposite end portions of the braid 12. Then, as shown in Fig. 8, the flex 11 having the lubricating layer 16 preformed thereon is inserted into the sheath 15. The length of the flex 11 in its natural condition is substantially equal to the length of the braid 12. The insertion of the flex 11 into the sheath 15 will now be described in detail. First, there is prepared a core member 30 having an outer diameter smaller than the inner diameter of the flex 11. The outer diameter of the core member 30 is smaller than the inner diameter of the sheath 15, and the difference between this outer diameter and this inner diameter is greater than the double of the thickness of the flex 11.

The flex 11 is fitted on the core member 30, and is twisted into a smaller diameter, so that the flex 11 is brought into contact with the outer peripheral surface of the core member 30. This reduced-diameter flex 11 has a length generally equal to its length in its natural condition, and the pitch of the turns of the reduced-diameter flex 11 becomes smaller. In this condition, the opposite ends of the flex 10 are fixed to the outer peripheral surface of the core member 30 by screws 31. Then, the core member 30 with the flex 11 is inserted into the sheath 15. Then, the fixing of the opposite ends of the flex 11 to the core member 30 is released by removing the screws 31, so that the flex 11 is increased in diameter to be brought into contact with the sheath 15 under a small pressure. In this manner, the flex 11 can be easily inserted into the sheath 15. Thereafter, the core member 30 is moved axially and is withdrawn from the flex 11 and the sheath 15.

Then, the opposite end portions of the braid 12 are soldered respectively to the outer peripheral surfaces of the opposite end portions of the flex 11, and the opposite end portions of the flex 11 as well as the opposite end portions of the braid 12 are soldered respectively to the rings 17 and 18 shown in Fig. 2.

In the above flexible tube-producing method, after the sheath 15 is formed, the flex 11 is inserted thereinto, and therefore the flex 11 is positively prevented from being bonded to the sheath 15 by the adhesive. Further, since the adhesive reaches the outer peripheral surface of the core member 20 and is cured there, the inner peripheral surface of the adhesive layer 14 is smooth. Therefore, the turns of the flex 11 can be smoothly moved relative to the sheath 15.

Since the core member 20 is to be dissolved later, there is no need to form a layer of a non-adhesive nature on the outer peripheral surface of the core member 20. Therefore, the adhesive is allowed to be satisfactorily bonded to the core member 20, thereby forming a smoother surface. On the other hand, the lubricating layer 16 is formed on the outer peripheral surface of the flex 11. The lubricating layer 16 does not need to have the function of preventing the adhering of the adhesive thereto, and therefore the material for the lubricating layer 16 can be selected, only taking its frictional resistance into consideration. Therefore, the lubricating layer 16 can be made of a material having a very low frictional resistance. For example, the lubricating layer 16 can be made of molybdenum disulfide or ceramics. As a result, the frictional resistance between the inner peripheral surface of the adhesive layer 14 (i.e., the inner peripheral surface of the sheath 15) and the flex 11 is very low, and therefore the turns of the flex 11 can be more smoothly moved relative to the sheath 15. As a result, the bending resistance of the flexible tube 10 can be reduced to a very small level.

When the adhesive is to be impregnated into the braid 12, it is not necessary at all to consider the adhering of the adhesive to the outer peripheral surface of the flex 11. Therefore, the adhesive can be sufficiently impregnated into the braid 12, and the strength of bonding between the braid 12 and the resin layer 13 can be increased. This prevents a premature rupture of the resin layer 13 which would otherwise be caused by the separation of the braid 12 from the resin layer 13 due to repeated bending of the flexible tube 10.

The present invention is not to be restricted to the above embodiment, and various modifications can be made.

The braid may be made solely of chemical fibers, or may be made of a combination of chemical fibers and metal wires.

The core member to be inserted into the braid for assembling the sheath may be made of a glass material dissolvable in an alkali solution. Also, this core member may be made of any other suitable dissolvable material than a glass material.

The core member to be inserted in the braid may be removed from the sheath by moving the core member axially relative to the sheath. In this case, the core member is in the form of a bar or a pipe made of stainless steel, brass, aluminum or the like. Also, in order to reduce a frictional resistance between the core member and the braid, a layer of a non-adhesive nature is preferably preformed on the outer periphery of the core member. Examples of such non-adhesive material include PTFE (polytetrafluoroethylene) and FEP (tetrafluoroethylene-hexafluoropropylene copolymer).

The following modified methods may be used for forming the resin layer. These method are similar to the above embodiment in that the braid is fitted on the core member, and the adhesive is impregnated in the braid and is cured to form the adhesive layer.

A tube of a thermoplastic resin is heated and softened, and is increased in diameter by an air pressure. This tube is used instead of the heat-shrinkable tube of the above embodiment. The thus enlarged-diameter tube is fitted on the braid 2, and then the tube is heated at a temperature higher than the temperature of the heat used for the above diameter-increasing step, and then the tube is returned to its initial diameter to be brought into intimate contact with the braid. At this time, the braid and the tube are bonded together by the adhesive.

According to another modified method, a tube of a thermoplastic resin is fitted on the braid, and this tube is heated and softened, and in this condition pressure is applied to the outer peripheral surface of the tube by rollers or the like, thereby bringing the tube into intimate contact with the braid.

According to a further modified method, a molten resin is applied to the outer peripheral surface of the braid by extrusion, and then the molten resin is cured to form the resin layer.

According to a further modified method, a resin in a liquid state is applied to the braid by dipping, and then the resin is dried and cured to form the resin layer. In this dipping method, in the case where the adhesive is not used, the braid is embedded in the inside portion of the cured resin layer, thereby providing the flexible tube 10A shown in Fig.4. With this method, the braid 12 and the resin layer 13A can, of course, be firmly joined together. The inner peripheral surface of the resin layer 13A is smooth, and part of the resin layer 13A will not intrude into the spaces between the turns of the flex 11, thereby ensuring a smooth movement of the turns of the flex 11 relative to the sheath 15A.

Particularly where the dipping method is used, it is necessary that at least one end of the glass pipe used as the core member should be closed so as to prevent the molten resin from intruding into the glass pipe.

The methods in which the dissolvable core member is removed from the sheath by dissolving this core member can be used for producing the type of flexible tube having no flex.

## Claims

1. A method of producing a flexible tube comprising the steps of :
   (a) fitting a braid on an outer periphery of an elongated core member of a cylindrical shape;
   (b) forming a soft resin layer on an outer periphery of said braid, and joining said resin layer and said braid together to form a sheath;
   and
   (c) removing said core member from said sheath ;
   CHARACTERIZED by the step of inserting a flex (11) into said sheath (15 ; 15A) after said step (c), said flex being in the form of a spirally-wound metal strip.

2. A method according to claim 1, in which said core member (20) is made of a dissolvable material, said core member being removed from said sheath (15 ; 15A) by dipping said core member in a dissolving solution (22).

3. A method according to claim 1, in which said step (d) comprises winding said flex (11) on a second core member (30) so as to bring said flex into a diameter smaller than the diameter of said flex in its natural condition, said second core member having an outer diameter smaller than the inner diameter of said sheath (15 ; 15A), and the difference between said outer diameter and said inner diameter being greater than the double of the thickness of said flex ; subsequently fixing opposite ends of said flex to said second core member; subsequently inserting said flex into said sheath; and subsequently releasing the fixing of the opposite ends of said flex to said second core member; and subsequently removing said second core member from said sheath.

4. A method of producing a flexible tube comprising the steps of :
   (a) fitting a braid on an outer periphery of an elongated core member of a cylindrical shape; and
   (b) forming a soft resin layer on an outer periphery of said braid, and joining said resin layer and said braid together to form a sheath;

   CHARACTERIZED in that said core member (20) is made of a dissolvable material ; and that said core member is removed from said sheath (15) by dipping said core member in a dissolving solution (22).

5. A method according to claim 4, in which said core member (20) is made of a glass material.

6. A method according to claim 4, in which said core member (20) is in the form of a pipe.

7. A method according to claim 6, in which opposite ends of said core member (20) are beforehand closed, said opposite ends of said core member being cut to be opened after the formation of said sheath (15) and before the removal of said core member from said sheath.

# Fig.1

# Fig.2

# Fig.3

# Fig.4

## Fig.5

## Fig.6

## Fig.7

## Fig.8